# EUROPEAN PATENT APPLICATION

(11) **EP 2 692 384 A2**
(43) Date of publication of application: **05.02.2014**
(21) Application number: 13000649.7
(22) Date of filing: 07.02.2013
(51) Int. Cl.: A61M 16/00, A61M 16/12, A61M 16/20

(54) **Gas administration device, especially resuscitation device**

(30) Priority: 31.07.2012 EP 12005569
(71) Applicant: Linde Aktiengesellschaft, 80331 München (DE)
(72) Inventor: Weiszl, Gunther, 2513 Möllersdorf (AT)
(74) Representative: Kasseckert, Rainer

(57) **Abstract**

The present invention relates to a Resuscitation device (100) comprising a compressible chamber (110) for providing a tidal volume of oxygen-containing gas to a patient, with an inlet (111) for admission of the oxygen-containing gas into the compressible chamber (110), and an outlet (112) for directing the oxygen containing gas to a patient, the compressible chamber being compressible by means of a lever mechanism (120) comprising at least one pivotable lever (122), the lever mechanism (120) being actable by means of a cam mechanism (130) comprising a cam pivotable about a cam axis (132), wherein the tidal volume of the compressible chamber is adjustable by means of moving the compressible chamber and/or by moving the cam axis (132) of the cam (130) with respect to the at least one lever (122), and/or by moving the lever (122) with respect to the-compressible chamber.

## Description

The present invention concerns a gas administration device, especially a resuscitation device, according to the preamble of claim 1.

### Background of the invention

Gas administrators, especially resuscitators, serve to supply air or oxygen to a patient. Typical examples are bag valve mask - type resuscitators. Furthermore, pump-like resuscitators and pressure-limited resuscitators are known.

In the field of neonatal resuscitators, it is important to avoid damaging a patient's lungs by delivering a too large volume of air resulting in barotraumata.

WO 2008/147229 A1 describes an electrically operable resuscitation device comprising a pump including a rigid cylinder including at least one gas inlet and at least one gas outlet, a piston to travel in said cylinder, and at least one valve, the or each valve configured to allow gas to be displaced into said cylinder through at least one gas inlet during at least one of a first stroke direction and second stroke direction of said piston in said cylinder, and for allowing gas displaced through said at least one gas outlet during an opposite of said at least one of the first stroke direction and second stroke direction of said piston in said cylinder. This mechanism is considered complicated, and not easily adjustable to specific needs.

The object of the invention is thus to provide a resuscitation device, which is easy to operate and modify according to different conditions and requirements.

### Summary of the invention

This object is achieved by a device comprising the features of claim 1.

According to the invention, there is suggested a gas administration device, especially a resuscitation device, with which the tidal volume, breathing frequency and maximum pressure delivered to a patient can easily be modified, thus avoiding excessive volumes of gas being delivered to a patient. At the same time, the resuscitation device according to the invention allows a simple modelling of a clinically desired or validated inhalation profile by means of providing a corresponding cam profile.

Advantageous embodiments of the invention are the subject-matter of the dependent claims.

According to a preferred embodiment, the position of the compressible chamber with respect to the at least one lever is adjustable by means of a positioning mechanism, especially an elevating screw mechanism. Such a mechanism allows a simple and reliable positioning of the compressible chamber relative to the lever mechanism, whereby the tidal volume can be easily adjusted by using pusher devices for adjusting the position of the compressible chamber.

Preferably, the lever mechanism and/or the bellows comprises a retaining mechanism, especially a return spring mechanism. By means of such a retaining mechanism it is ensured that compressible chamber and lever as well as lever and cam will always have physical contact to each other and will always be positioned in accordance to the cam, ensuring that the position of the cam controls the travel of the compressible chamber.

Preferred embodiments of such retaining means comprise at least one spring acting on the lever and/or the compressible chamber, or an elastic component. It is especially possible to inherently provide the compressible chamber with such spring or elastic characteristics.

The bellows can be provided as a "self inflating bag", i.e. essentially a bag with sufficient compliance. In case of insufficient compliance, this can be enhanced by providing additional spring means along the bellows axis or, for example, by means of a torsion spring in the axis of rotation of the lever. These means can act on the upper side of the bellows and ensure that bellows, lever and cam contact each other at any time, i.e. that the opening or momentary volume of the bellows is always defined by the position of the cam.

Advantageously, the cam is provided with a cam profile, by means of which a clinically validated or desired inhalation profile can be generated. Such cams with desired cam profiles can be provided in an interchangeable manner. For example, a cam can be provided to be manually exchangeable. Also, it is possible to provide a number of different cams with corresponding cam profiles, which can automatically (electronically) be chosen by a user in order to choose a desired inhalation profile.

Expediently, a bearing mechanism can be provided on the at least one lever of the lever mechanism, on which the cam acts. This can minimize any frictional effects, thus leading to a longer lifetime and less wear and tear, as well as increased reliability of the resuscitation device.

According to a preferred embodiment, for which independent protection is sought, there is provided a PEEP-mechanism at an outlet side of the compressible chamber.

Such a PEEP device can especially be provided with a novel membrane interacting with an adjustable spring, as described in detail with reference to the figures.

According to a further modification, for which independent protection is also sought, there is provided a dosage means for additional dosage of oxygen into the gas, especially air, delivered by the compressible chamber. Such a means can especially comprise a flow regulator for providing constant flow of additional oxygen, especially in order to keep a FIO₂ (fraction of inspired oxygen) value constant. Expediently, such a dosage means is provided between the compressible chamber and the PEEP-mechanism.

Furthermore, according to a further embodiment, for which independent protection is sought, there is provided a means for buffering a gas volume administed to a patient, provided at the outlet side of the compressible chamber, especially between the compressible chamber and the PEEP-mechanism. By providing such a buffering means, for example sudden pressure peaks, which might damage or discomfort a patient, can be avoided, without having to let gas escape into the surroundings.

### Brief description of the drawings

A preferred embodiment of the invention will now be described with reference to the accompanying drawings. Herein,
- Fig. 1: shows a schematic side view of a preferred embodiment of a resuscitator according to the invention,
- Fig. 2: shows a front view of a PEEP-valve as usable according to the preferred embodiment of Figure 1,
- Fig. 3: shows in a schematic side view, a further embodiment of a PEEP mechanism as usable according to the invention,
- Fig. 4: shows in a schematic side view, a further embodiment of a resuscitator according to the invention, and
- Fig. 5: shows in a schematic side view, a further embodiment of a resuscitator according to the invention.

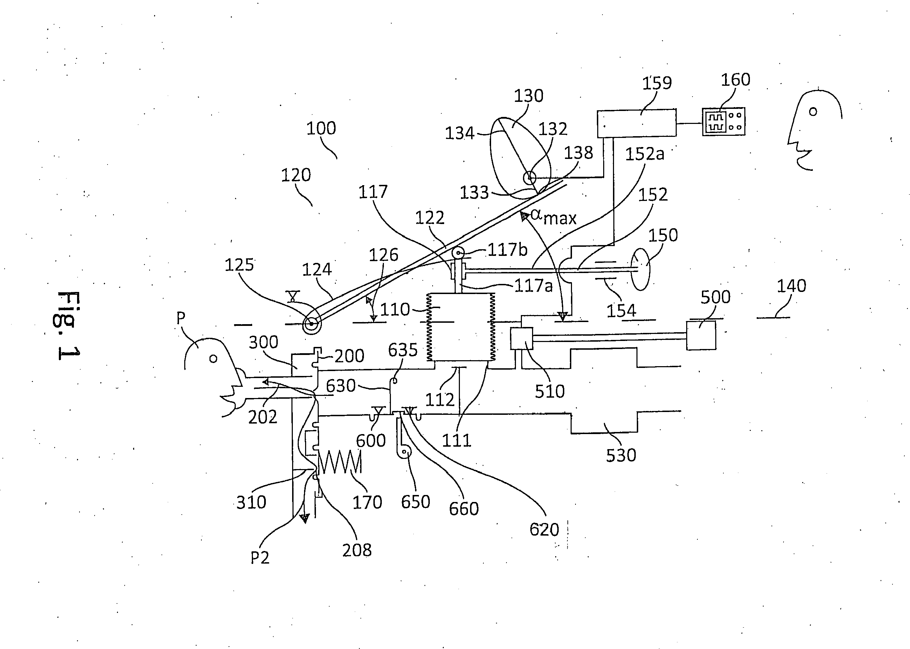

Fig. 1 shows a preferred embodiment of a resuscitator according to the present invention, generally denoted 100.

The resuscitator 100 comprises a compressible chamber in form of a bellows 110. The bellows 110 comprises an inlet 111 for admission of gas, i.e. air or oxygen-containing gas, into the bellows 110. It further comprises an outlet 112 for directing gas to a patient P. Inlet 111 and outlet 112 are provided with corresponding check valves, as is well known in the prior art (not shown).

The bellows 110 is compressible by means of a lever mechanism 120 comprising a first lever 122 and a return spring 124 acting as retaining mechanism.

The lever 122 is pivotable about a pivot axis 125, in the vicinity of which the return spring 124 can be positioned, for example in case of usage of a torsion spring as return spring 124.

By means of a pivoting movement about the pivot axis 125 the lever 122 can rotate in a clockwise and anti-clockwise direction, as indicated by double pointed arrow 126. This pivoting movement of the lever 122 is generated by rotation of a cam 130, which rotates about a cam axis 132. It is especially preferred that the cam rotates about cam axis 132 in a steady manner, i.e. with constant angular velocity. The profile of the cam is designed so that a desired clinically valid inhalation profile can be achieved. Also, the profile of the cam 130 determines the I/E-ratio (time ratio of inhalation to exhalation), for example 1:1.5. By arresting the cam e.g. in its upper position, i.e. the position shown in figure 1, the steady rotation can be interrupted (i.e. by holding the cam in this position for a certain time), whereby exhalation intervals can be increased.

The cam 130 is driveable by a drive (not shown), that is controlled to accomplish certain RPMs. Preferably a computing device 159 is provided to allow the user to select the parameters and display the data via a graphical user interface 160.

It is also advantageous to provide disposables such as standard bag-valve-mask or BVM-solutions. Especially, inhalation/exhalation valves manifolds, tubings or masks used can be provided in such a way.

The lever 122 is pivotable about axis 125 and thus positionable at an angle relative to an (imaginary) horizontal axis 140. The bellows 110 is moveable/displaceable along or parallel to this axis 140.

In Fig. 1, the lever 122 is shown at a maximum angle αₘₐₓ relative to the horizontal axis 140. This angle αₘₐₓ corresponds to a minimum radius 133 of the cam 130 contacting the lever 122. The maximum radius of the cam 134 corresponds to a minimum angle αₘᵢₙ of the lever (not shown). Advantageously, the angle αₘᵢₙ corresponds to the axis140. i.e. the lever 122 is pivotable between the position shown in figure 1 and axis 140. As will immediately be seen, the lever 122 moves backwards and forwards (clockwise and anti-clockwise) between αₘₐₓ and αₘᵢₙ (preferable 0°, corresponding to axis 140), corresponding to the angular position of cam 130.

The bellows 110 is, as mentioned, displaceable along horizontal axis 140. Such a displacement can be achieved by a corresponding drive 150, which drives a (positioning mechanism) 152, preferably an elevating screw mechanism comprising a thread rod 152a. Expediently, this thread rod interacts withy threading forming a guide (denoted as 154 in Fig. 1). The side of the bellows interacting with the lever 122 can be provided with an interaction mechanism 117, for example a holder 117a supporting a bearing 117b, the bearing contacting the lower side of the lever 122. In a preferred embodiment also, a bearing mechanism 138 or other friction reduction means can be provided on the area of the lever 122 with which the cam interacts. Instead of such an elevating screw mechanism, drive means can also be provided in form of, for example, a simple linear actuator or a pusher mechanism.

Depending on its position along axis 140, the bellows 110 will be more or less compressed by lever 122 as it moves e.g. from αₘₐₓ to αₘᵢₙ, for example αₘᵢₙ = 0°. For example, in the position shown, the bellows will be compressed from an intermediate maximum volume, as for example compared to a position further to the right, i.e. nearer to drive 150, where the maximum volume of the bellows will be larger. A position of the bellows further to the left, i.e. nearer to pivot axis 125, will lead to a smaller maximum volume. In other words, the further towards the pivot axis 125 the bellows 110 is moved, the smaller the tidal volume, which the bellows 110 administers (i.e. the difference between maximum and minimum volume) becomes.

The residual volume of bellows 110, i.e. the volume of gas remaining in the bellows at its minimum volume or in the position αₘᵢₙ =0° of the lever, is not dependent on the position of the bellows 110 along the axis 140.

The larger the tidal volume, the higher also the flow rate of gas discharged or administered by the bellows 110.

Be it noted that the acutation of the lever 122 can be performed by a driven cam, as described, or, additionally or alternatively, by manually driving the cam, or even by directly actuating the lever, i.e. without making use of the cam.

Advantageously, additionally or alternatively to the movement of the bellows along axis 140, it is also possible to move the position of cam 130, i.e. its axis of rotation 132. Hereby also, the tidal volume of the bellows can be manipulated in a desired way. As described with respect to Figure 1, the bellows 110 is driven by lever 122 being rotatable about pivot axis 125.

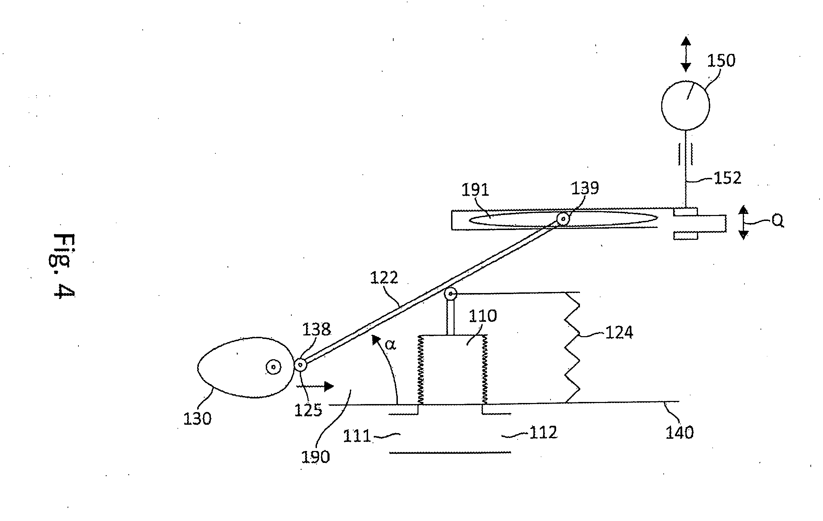

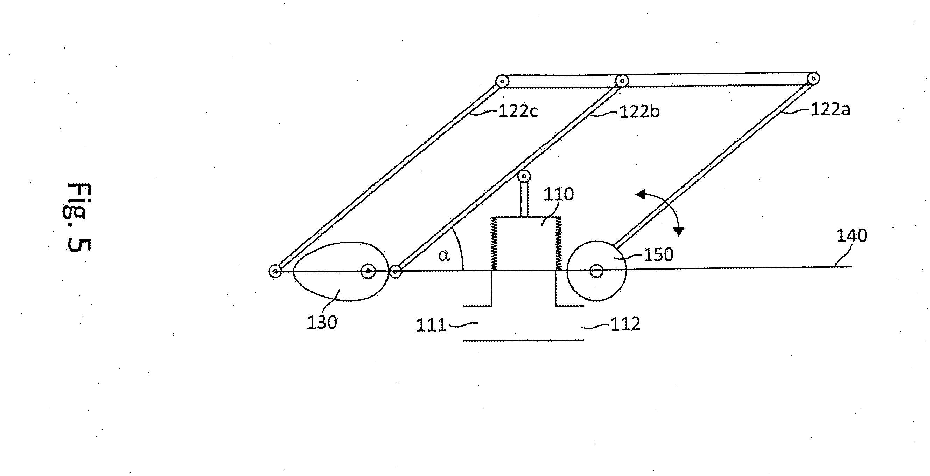

According to further embodiments, shown in Figures 4 and 5, it is also possible to drive the bellows by means of a lever performing a translational movement along axis 140. For example, as shown in Figure 4, the cam 130 is arranged to the left of the lower end 138 of lever 122. Through actuation by cam 130, this end 138 of lever 122 performs a translational movement along a first guide 190 parallel to axis 140. The upper end of lever 122 is designated 139. It is correspondingly moveable (translatable) in a guide 191, which extends parallel to guide 190 and thus parallel to axis 140. Thus; by interaction with the cam 130, lever 122 will perform a translational movement backwards and forwards along axis 140, which will lead to a corresponding deflation and inflation of bellows 110. A spring 124 serves to expand the bellows 110 while lever 122 is moving away from bellows 110, i.e. to the left in Figure 4. The angle α in Fig. 4, which defines the inclination of lever 122 with respect to axis 140, is adjustable by means of a shifting mechanism for guide 191, by means of which guide 191 can be moved towards or away from axis 140, as symbolized by double pointed arrow Q. The shifting mechanism can comprise a wheel 150 and a pusher 152 acting on guide 191.

A similar mechanism employing parallel levers is schematically shown in Figure 5, where again similar or identical components are designated with the same reference numerals. Three interconnected parallel levers 122a, 122b, 122c are provided, which can be jointly moved along axis 140 by means of cam 130. Inclination angled α can be set by means of an adjustment mechanism comprising a wheel 150.

Hereby also, the tidal volume of the bellows can be manipulated in a desired way.

With the device according to the invention, e.g. tidal volumes, typical breathing patterns, breathing frequencies, ratio of I/E, minute volumes and pressure limitations can be set, modified and controlled. Furthermore, corresponding information can be presented to a patient or a user in an easily understandable way (graphic display). Critical situations, for example elevated pressure, can, for example, be indicated by means of an acoustical or an optical alarm. Other breathing parameters, for example minute volumes that result from the set parameters can be calculated and displayed to a user: The resulting breathing pattern that will be administered to the patient can be graphically displayed.

Various modes of operation are contemplated. For example, a fully automatic mode, e.g. comprising predetermination of tidal volume and breathing frequency, can be set. Also, half automatic modes can be advantageous, in which, for example, individual breaths of air can be delivered/simulated by for example pressing a control device (button). The user interface they always display the current breathing parameters that result from a manual operation, e.g. by counting the control device knob actuations or the rotations of the cams.

Also, manual modes of operation are possible, for example in case of lacking knowledge of the device by a user. Taking into account that this device is intended as life saving device, certain events like lacking electricity (lacking mains or battery) or device failures can be mitigated by a manual backup option and the therapy can be continued without harming the patient. The device according to the invention ensures predetermined maximum tidal volumes and maximum pressures in all modes of operation. A manual operation can be detected for example when a contact between the cam 130 and the leaver 122 is lost, and a second contact in the 0°-position shows that the user is effecting a manual resuscitation. In this case also, breathing parameters could be calculated by the device.

The device according to the invention can be advantageously provided together with a "PEEP"-mechanism (PEEP: positive end expiratory pressure).

Positive End-Expiratory Pressure (PEEP) designates a positive pressure within the lungs, which can be generated during resuscitation. Various medical conditions can be alleviated by providing such a positive pressure. It is advantageous to be able to modify a PEEP generated or provided by a resuscitation device, in order to accommodate various medical conditions.

Figure 1 also shows how the inhalation/exhalation valve assembly shown comprises a PEEP valve without adding further components to the disposable circuitry. A valve manifold 200 comprising various functional sections is provided in a case 300 (schematically shown), both assembled to one physical, disposable component. This assembly is positioned between the outlet side 112 of the bellows 110 and the patient P.

Valve manifold 200 comprises an opening 202 within a first section 201, which, during reduction of the bellows volume, i.e. during inhalation, will allow air to pass from outlet 112 to patient P.

During expiration, air can be discharged by means of a second section 208 of the valve manifold 200, which is urged against an opening 310 of the disposable case 300 by means of a force actuator, preferably a spring 170. This second section 208 is delimited by a lip 210. The force acting against this second section 208 (i.e. generated by spring 170) is adjustable. The force is relevant for the pressure that needs to be applied on the second section 208, representing the PEEP. This passage of air is symbolized by curved arrow P2. As can be seen from Fig. 1, the lip 210 surrounds said opening 310. By adjusting the tension of the spring 170, the PEEP can thus be controlled according to the needs of a patient via the user interface of the device.

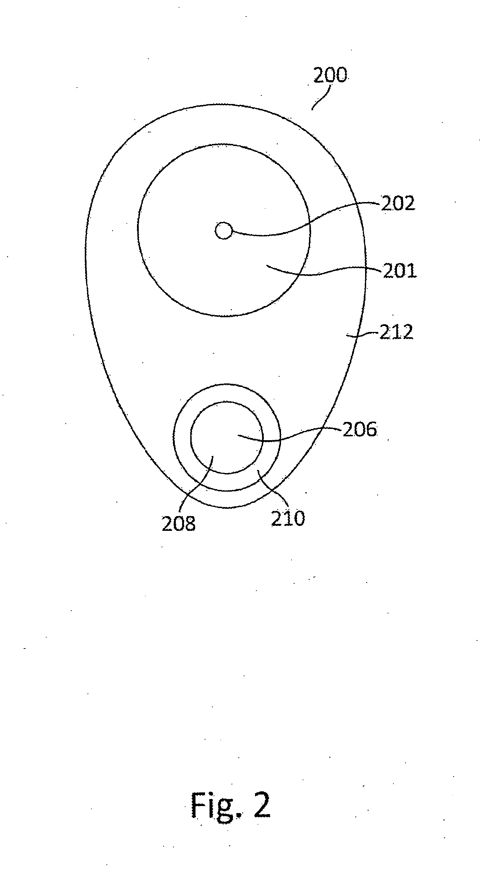

Fig. 2 shows the membrane 200 as used in the embodiment of Fig 1. The membrane may be preferably produced as a single part comprising of an elastomeric material like silicone. While prior art incorporates only components 201 and 202, this embodiment according to the invention additionally also comprises components 206, 208, 210.

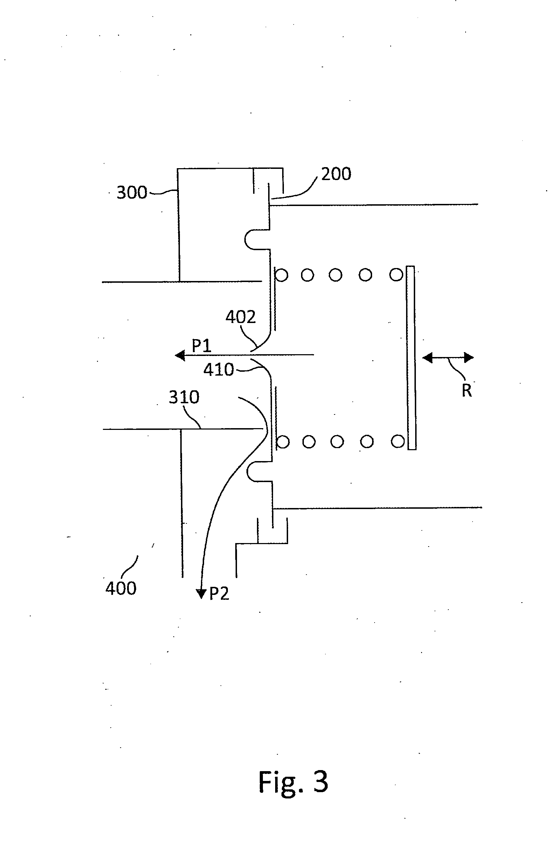

A further embodiment of a PEEP-valve 400 and its positioning in a disposable case is shown in Fig. 3. Here, the lip, designated 410, surrounds the valve opening, designated 402. The PEEP-valve 400 covers an opening 310 of the disposable case 300. Again, inspiration can be effected through valve opening 402 (arrow P1). Expiration is effected through opening 410 (arrow P2). Again, PEEP is adjustable by means of spring 420 (symbolised by double pointed arrow R).

A further advantageous embodiment of the invention comprises the additional dosage of oxygen to inlet air, i.e. referring to Fig. 1 to air entering the system via inlet 111.

Such an additional dosage of oxygen can be affected on the inlet side of the compressible chamber, i.e. in connection with passage 111, as will be explained in the following: In order to add oxygen with a desired FIO₂ value, it is required to provide an oxygen source 500 with a specific pressure range, for example between 2.8 bar and 6.0 bar. According to the present invention, the resuscitation device provides information regarding the tidal volume and the breathing frequency, and can thus determine the minute volume. Consequently, it can calculate the necessary oxygen flow to be added in order to provide a specific FIO₂, for example by means of an oxygen flow regulator 510. Preferably, only one additional pneumatic element is necessary, for example an electrically driveable mechanical flow control device. To achieve this, it is only necessary to provide a specific buffer volume 530 upstream of the air intake, in order to adapt a constant additional dosage to a variable discharge. Thus, as a person skilled in the art will realize, this constitutes a similar principle as in connection with a free flow scavenger, which is, however, utilized on the inhalation side.

A further advantageous embodiment of the invention concerns the buffering of the tidal volume. Typical prior art allows relief of gas to ambience, which protects the patient, but does not allow any conclusions how much gas has entered the patient. In case tidal volume is an important parameter, one would like to ensure that patient receives the desired amount while being protected from excessive pressure.

Thus, in order to minimize any risk of a tidal volume being administered to a patient too quickly, or situations, in which the pressure with which the patient is confronted exceeds an upper limit, it should be possible to limit the administered pressure by discharging air not to the patient, but to the environment or a buffer volume. Alternatively the current embodiment may still provide options to manually select between pressure relief and pressure buffer.

In this connection of buffering the gas, there are multiple options, such as:
- Stopping the therapy and require action (modification of the parameters) by the operator
- Maintain the currently targeted exhalation time: by stopping the cam by the increase of inhalation time (caused by the time the buffer discharges),
- Maintain the currently targeted minute volume (if possible): by reducing the I/E ratio to allow longer inhalation times causing lower inhalation flows,
- Maintain the currently targeted I/E: by reducing the breathing frequency
- Maintain the currently set parameters but displaying the alarm on the user interface.

It is especially advantageous to discharge the excess air into a buffer volume, which can be utilized at a later time. This will now be further explained with reference to figure 1.

A membrane 600 can be provided in outlet 112, preferably between compressible chamber 110 and valve manifold. It can be provided as a membrane-like section of the wall of outlet 112. Preferably, it abuts against stops 620. In case the pressure in outlet 112 exceeds a desired or predetermined value, the membrane will expand away from stops 620, thus enlarging the volume of outlet 112. In order to be able to adjust the pressure at which this effect occurs, the force load on the membrane is provided with a force actuator 630, for example a spring, which extends for example from the centre of the membrane 600 to a guide 635, about which it can be wound. Thus, by winding the actuator 630 about axis 635, the tension of membrane 600 can be adjusted. The characteristic curve of this elastic spring is preferably soft, i.e. when the spring is expanded, this can occur without significant changes to the forces acting, enabling the buffer to be filled at fairly constant pressure. In an alternative embodiment, the actuator 630 can be adjusted linearily instead by an axis 635.

A pressure for ensuring an opening of this membrane can be defined as spring force multiplied with membrane area. A handwheel, by which the spring can be turned about guide or axis 635 can include a scale for showing the set pressure. A position feedback with the handwheel can inform the system as a whole as to the set opening pressure. In case of an additional PEEP-system, it must be ensured that the opening pressure lies above PEEP. Otherwise, air would constantly be discharged. Thus, according to the invention, the adjusting mechanisms may be adapted to one another. The abutment of the membrane may be detected by the device, e.g. by identification of the contact between 620 and 600.

In order to allow this pressure relief there may be provided a check valve type component 660 which is pushed open by an actuation knob 650 right after the membrane 600 lifts off from the stops 620. The actuation knob 650 switches between a disabling position (as shown in Fig. 1) and an enabling position, e.g. by rotating pins towards the valve 620.. In the disabling position, no buffering of the volume of outlet 112 is possible, as component 650 abuts against membrane 600 or check valve 660 from the outside, relieving the volume. By moving or rotating component 650 away from the membrane or check valve, this abutment is cancelled, so that a volume increase can occur.

## Claims

1. Gas administration device, especially resuscitation device (100) comprising a compressible chamber (110) for providing a tidal volume of oxygen-containing gas to a patient, with an inlet (111) for admission of the oxygen-containing gas into the compressible chamber (110), and an outlet (112) for directing the oxygen containing gas to a patient, the compressible chamber being compressible by means of a lever mechanism (120) comprising at least one pivotable lever (122), the lever mechanism (120) being actable by means of a cam mechanism (130) comprising a cam pivotable about a cam axis (132),
**characterized in that**
the tidal volume of the compressible chamber is adjustable by means of moving the compressible chamber and/or by moving the cam axis (132) of the cam (130) with respect to the at least one lever (122), and/or by moving the lever (122) with respect to the compressible chamber.

2. Gas administration device according to claim 1, wherein the position of the compressible chamber (110) with respect to the at least one lever (122) and/or the lever (112) is adjustable by a positioning mechanism (152, 154).

3. Gas administration device according to claim 1 or 2, **characterized in that** the lever mechanism (120) comprises at least one retaining mechanism (124) interacting with lever (122).

4. Gas administration device according to any one of the preceding claims, **characterized in that** the cam (130) is provided with a cam profile by means of which a clinically validated inhalation profile can be generated.

5. Gas administration device according to any one of the preceding claims, further comprising a bearing mechanism (138) provided on the interface, on which the cam (130) acts.

6. Gas administration device, especially according to any one of the preceding claims, comprising a PEEP-mechanism (210, 310, 170) on the outlet side (112) of the compressible chamber (110).

7. Gas administration device, especially according to any one of the preceding claims, comprising a means for (510, 530) dosage of additional oxygen to the gas administered by the compressible chamber.

8. Gas administration device, especially according to any one of the preceding claims, comprising a means for (600, 620, 630, 650, 660) buffering a gas volume administered to a patient, provided at the outlet side (112) of the compressible chamber (110).
